# EUROPEAN PATENT APPLICATION

(11) **EP 3 662 859 A1**
(43) Date of publication of application: **10.06.2020**
(21) Application number: 18842382.6
(22) Date of filing: 20.07.2018
(51) Int. Cl.: A61B 34/37, B25J 3/00

(54) **MASTER OPERATION INPUT DEVICE AND MASTER-SLAVE MANIPULATOR**

(30) Priority: 31.07.2017 JP 2017147384
(71) Applicant: Medicaroid Corporation, Kobe-shi, Hyogo 650-0047 (JP)
(72) Inventor: ISHIHARA, Kazuki, Kobe-shi Hyogo 650-0047 (JP)
(74) Representative: Hoffmann Eitle
(86) International application number: PCT/JP2018/027391
(87) International publication number: WO 2019/026654

(57) **Abstract**

A remote control apparatus (200) disclosed herein is a master operation input device for operating a slave manipulator (300) having a surgical instrument as an end effector. The master operation input device includes: a master operation main body (210) arranged at a distal end of the master operation input device as viewed from a fixed end of the master operation input device; a first operating portion (220) and a second operating portion (230) which are supported on the master operation main body so as to be capable of pivoting with respect to the master operation main body to enable opening and closing operations; and a grip (240) configured to be attachable and detachable to and from a distal end of the master operation main body.

## Description

### TECHNICAL FIELD

The present invention relates to a master operation input device and a master-slave manipulator, and particularly relates to a master operation input device for operating a slave manipulator having a surgical instrument as an end effector, and a master-slave manipulator including the master operation input device.

### BACKGROUND ART

Master operation input devices for operating a slave manipulator having a surgical instrument as an end effector have been known. Such a master operation input device is described, for example, in U.S. Pat. No. 8,638,057 and Japanese Unexamined Patent Publication No. 2012-131014.

U.S. Pat. No. 8,638,057 discloses a master control console (i.e., a master operation input device) for operating a robotic surgical manipulator (i.e., a slave manipulator) having a surgical instrument as an end effector. The master control console is provided with a master grip for performing operations, such as opening/closing, rotating, and moving operations for the surgical instrument of the slave manipulator. The operator operates the master grip with the thumb and the forefinger.

The surgical assistant robots which actually operate in hospitals are the daVinci Surgical System of Intuitive Surgical, as described in the aforementioned U.S. Pat. No. 8,638,057. Thus, at present, many of the operators are used to the operation of such a master grip as described in the U.S. Pat. No. 8,638,057.

Japanese Unexamined Patent Publication No. 2012-131014 discloses a master operation input device operated with the thumb and the forefinger and having a grip gripped by fingers other than the thumb and the forefinger. The master operation input device disclosed in the U.S. Pat. No. 8,638,057 does not have a grip.

### CITATION LIST

### PATENT DOCUMENTS

Patent Document 1: U.S. Patent No. 8,638,057
Patent Document 2: Japanese Unexamined Patent Publication No. 2012-131014

### SUMMARY OF THE INVENTION

### TECHNICAL PROBLEM

However, it depends on the respective operator whether the operator feels it easy to operate the device with a grip or feels it easy to operate the device without a grip. That is, ease of operation (i.e., usability) of the master operation input device greatly depends on individual operators.

The present invention is therefore intended to provide a master operation input device, the usability of which is changeable according to an operator, and a master-slave manipulator.

### SOLUTION TO THE PROBLEM

A master operation input device according to a first aspect of the present invention is directed to a master operation input device for operating a slave manipulator having a surgical instrument as an end effector, the master operation input device including: a master operation main body arranged at a distal end of the master operation input device as viewed from a fixed end of the master operation input device; a first operating portion and a second operating portion which are supported on the master operation main body so as to be capable of pivoting with respect to the master operation main body to enable opening and closing operations; and a grip configured to be attachable and detachable to and from a distal end of the master operation main body.

A master-slave manipulator according to a second aspect of the present invention includes: a slave manipulator having a surgical instrument as an end effector and having a plurality of joints; the master operation input device according to the first aspect of the present invention; and a controller which performs control to drive the plurality of joints of the slave manipulator, based on an operation of the master operation input device by an operator.

### ADVANTAGES OF THE INVENTION

The present invention is capable of changing usability according to an operator.

### BRIEF DESCRIPTION OF THE DRAWINGS

FIG. 1 is a diagram schematically illustrating a master-slave manipulator according to first to third embodiments.
FIG. 2 is a block diagram illustrating a control configuration of a master operation input device according to the first to third embodiments.
FIG. 3 is a diagram illustrating a perspective view of an operating handle of the master operation input device according to the first embodiment.
FIG. 4 is a diagram illustrating an exploded perspective view of the operating handle of the master operation input device according to the first embodiment.
FIG. 5 is a diagram illustrating a plan view of the operating handle of the master operation input device according to the first embodiment.
FIGS. 6A, 6B, and 6C are diagrams respectively illustrating side views of first, second, and third examples of the operating handle of the master operation input device according to the first embodiment.
FIG. 7 is a diagram illustrating a side view of a first example of a grip of the master operation input device according to the first embodiment.
FIG. 8 is a diagram illustrating a side view of a second example of the grip of the master operation input device according to the first embodiment.
FIG. 9 is a diagram illustrating a side view of a third example of the grip of the master operation input device according to the first embodiment.
FIG. 10 is a diagram illustrating a side view of a fourth example of the grip of the master operation input device according to the first embodiment.
FIG. 11 is a diagram illustrating a perspective view of an operating handle of the master operation input device according to the second embodiment.
FIG. 12 is a diagram illustrating an exploded perspective view of the operating handle of the master operation input device according to the second embodiment.
FIG. 13 is a diagram illustrating a perspective view of an operating handle of the master operation input device according to the third embodiment.
FIG. 14 is a diagram illustrating an exploded perspective view of the operating handle of the master operation input device according to the third embodiment.

### DESCRIPTION OF EMBODIMENTS

The embodiments will be described in detail below with reference to the drawings.

### [First Embodiment]

### (Configuration of Master-Slave Manipulator)

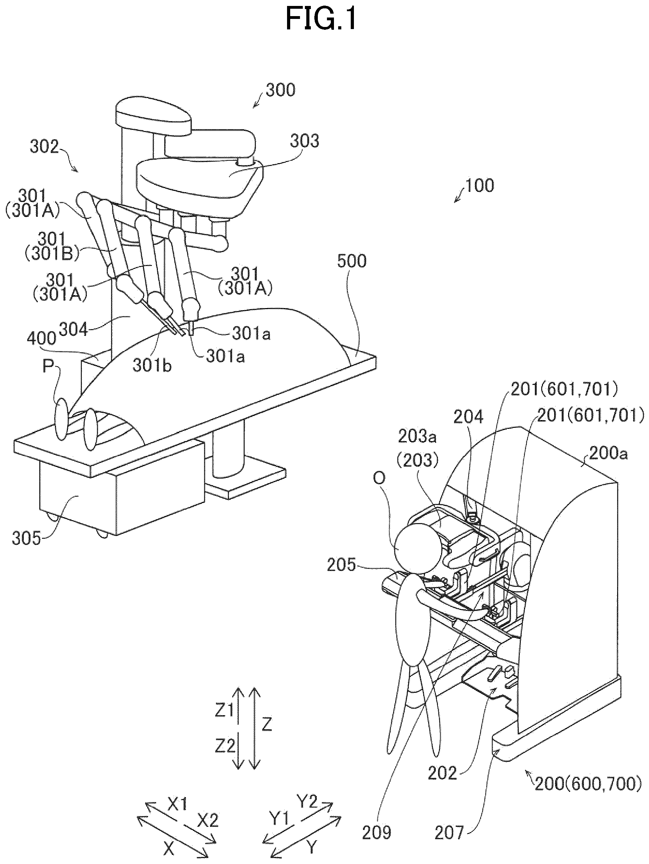

Configurations of a master-slave manipulator 100 according to the first embodiment will be described with reference to FIGS. 1 to 10.

As illustrated in FIG. 1, the master-slave manipulator 100 includes a master operation input device 200, a slave manipulator 300, and a controller 400.

The master operation input device 200 is configured to remotely control the slave manipulator 300. When an operator O, who is a surgeon, enters a movement type instruction to be executed by the slave manipulator 300 to the master operation input device 200, the master operation input device 200 transmits the movement type instruction to the slave manipulator 300 via the controller 400. The slave manipulator 300 then operates a surgical instrument gripped by a surgical manipulator 301, which will be described later, or medical equipment in response to the movement type instruction transmitted from the master operation input device 200. A minimally invasive operation is performed in this manner.

The slave manipulator 300 constitutes an interface through which a surgery is performed on a patient P. The slave manipulator 300 is arranged beside a surgical table 500 on which the patient P lies. The slave manipulator 300 has a plurality of surgical manipulators (i.e., surgical arms) 301. One of the surgical manipulators 301 grips an endoscope 301b, which is medical equipment, and the other surgical manipulators 301 grip surgical instruments (instruments 301a). That is, the slave manipulator 300 has surgical instruments and medical equipment as end effectors. The surgical manipulators 301 which grip the surgical instruments (instruments 301a) function as instrument arms 301A. The surgical manipulator 301 which grips the endoscope 301b functions as a camera arm 301B. Each of the instrument arms 301A and the camera arm 301B are supported by the same platform 303. Each of the plurality of surgical manipulators 301 has a plurality of joints. A driving section including a servomotor and a position detector such as an encoder are provided for each joint. Each surgical manipulator 301 is configured to be controlled such that the surgical instrument or the medical equipment attached to the surgical manipulator 301 makes a desired movement in response to a driving signal given via the controller 400.

The platform 303 is supported by a positioner 302 placed on the floor of the operating room. The positioner 302 includes a column 304 having a lifting shaft adjustable in a vertical direction, and the lifting shaft is coupled to a base 305 having wheels and movable on the floor surface.

The instrument arm 301A detachably holds the instrument 301a, which is a surgical instrument, at its distal end portion. The instrument 301a includes a housing attached to the instrument arm 301A, and an end effector provided at a distal end portion of an elongated shaft. Examples of the end effector include, but are not limited to, a grasping forceps, scissors, a hook, a high-frequency knife, a snare wire, a clamp, and a stapler, and may include various types of treatment tools. In a surgery using the slave manipulator 300, each of the instrument arms 301A is introduced into the body of the patient P through a cannula (a trocar) retained on the body surface of the patient P, so that the end effector of the instrument 301a is placed in the vicinity of a surgical site.

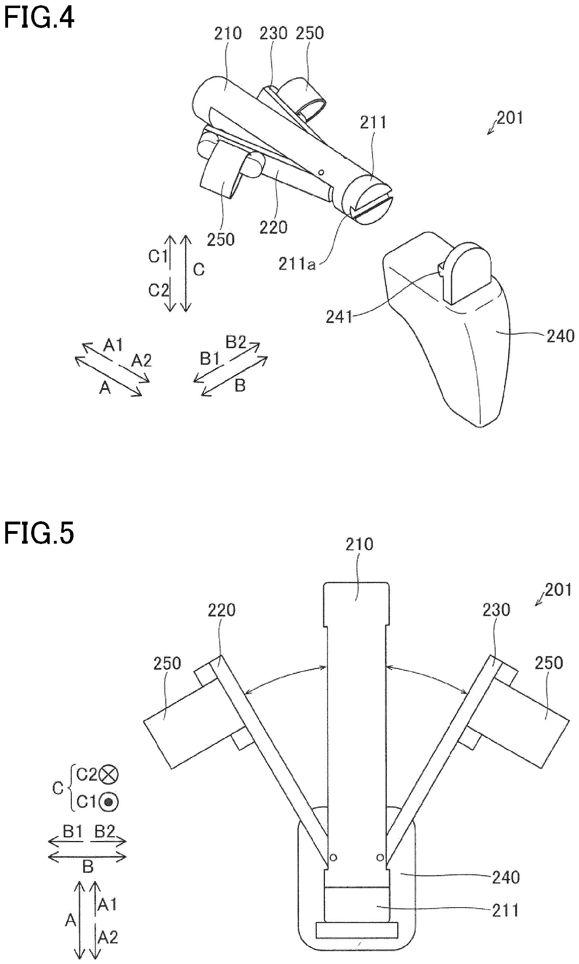

The endoscope 301b (see FIG. 4), which is the medical equipment, is detachably attached to a distal end portion of the camera arm 301B. The endoscope 301b takes images in the body cavity of the patient P. The images taken are output to the master operation input device 200. Examples of the endoscope 301b include a 3D endoscope capable of taking three-dimensional images, or a 2D endoscope. In a surgery using the slave manipulator 300, the camera arm 301B is introduced into the body of the patient P through a trocar retained on the body surface of the patient P, so that the endoscope 301b is placed in the vicinity of a surgical site.

The master operation input device 200 constitutes an interface with the operator O. The master operation input device 200 serves as a device through which the operator O remotely controls the medical equipment or the surgical instrument gripped by the surgical manipulator 301. That is, the master operation input device 200 is configured to be capable of transmitting, to the slave manipulator 300, the movement type instruction that has been input by the operator O and that should be executed by the instrument 301a or the endoscope 301b, via the controller 400. The master operation input device 200 is installed beside the surgical table 500 so that the operator can check the condition of the patient P while operating the master, for example. The master operation input device 200 may be configured, for example, to wirelessly transmit the movement type instruction, and may be installed in a room different from the operating room where the surgical table 500 is placed.

The term "movement type" to be performed by the instrument 301a may refer to the type of movement (a series of positions and orientations) of the instrument 301a and the type of movement executed by the function of the respective instruments 301a. For example, if the instrument 301a is a grasping forceps, the movement type to be performed by the instrument 301a includes positions of rolling and pitching of a wrist of the end effector, and opening and closing jaws. If the instrument 301a is a high-frequency knife, the movement type to be executed by the instrument 301a may include vibration of the high-frequency knife, specifically, a current supply to the high-frequency knife. If the instrument 301a is a snare wire, the movement type to be performed by the instrument 301a may include tightening, and releasing from the tightening. In addition, the movement type may include a movement of burning off a target site of the surgery, using a bipolar or a monopolar to which an electric current is supplied.

Examples of the movement type to be performed by the endoscope 301b include positioning, and determination of the orientation, of the tip end of the endoscope 301b, or setting of zoom magnification of the endoscope 301b.

The master operation input device 200 has a cover 200a. The cover 200a is arranged to cover the lateral sides (in the X direction), the back side (in the Y2 direction), and the upper side (in the Z1 direction) of the master operation input device 200.

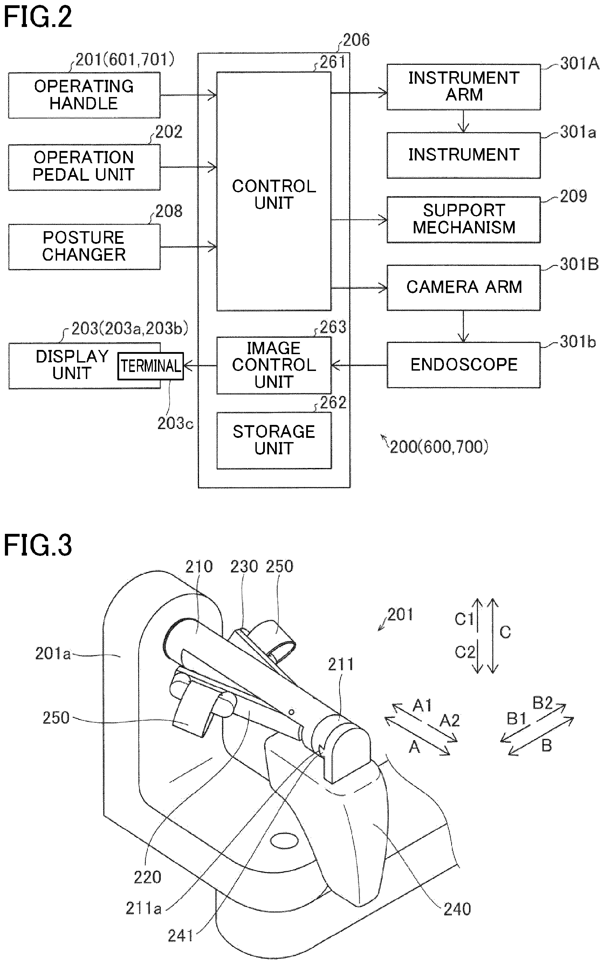

As illustrated in FIGS. 1 and 2, the master operation input device 200 includes an operating handle 201, an operation pedal unit 202, a display unit supporting arm 204 which supports a display unit 203, an arm rest 205 which supports the arms of the operator O, a control device 206, and a base 207. The master operation input device 200 includes a posture changer 208, and a support mechanism 209 which supports the operating handle 201 and the arm rest 205.

The operating handle 201 is provided to remotely operate the medical equipment or the surgical instrument gripped by the surgical manipulator 301. Specifically, the operating handle 201 accepts the operation conducted by the operator O to control the medical equipment (i.e., the endoscope 301b) or the surgical instrument (i.e., the instrument 301a). The operating handle 201 includes a pair of operating handles 201 along the X direction. Specifically, one of the pair of operating handles 201 toward the X2 direction (the right side) is operated by the right hand of the operator O, and the other one of the pair of operating handles 201 toward the X1 direction (the left side) is operated by the left hand of the operator O.

The operating handle 201 is attached to the support mechanism 209. Specifically, the operating handle 201 is connected to the support mechanism 209 through an arm portion 201a (see FIG. 3) having a plurality of joints, and is configured such that a distal end of the arm portion 201a is movable within a predetermined three-dimensional operation area with respect to the support mechanism 209. That is, the operating handle 201 is configured such that the distal end of the arm portion 201a is movable up and down directions (Z direction), the leftward and rightward directions (X direction), and the forward and backward directions (Y direction) with respect to the support mechanism 209. Each joint of the arm portion 201a is provided with a position detector (not shown) for detecting a positional relationship between the joints. The position detector is, for example, an encoder, a resolver, or a potentiometer, and detects the position of the operating handle 201 with respect to the support mechanism 209.

The master operation input device 200 and the slave manipulator 300 constitute a master-slave system in controlling the movements of the instrument arm 301A and the camera arm 301B. Specifically, the operating handle 201 serves as a controlling element (i.e., a master) in the master-slave system, and the instrument arms 301A which grip the surgical instruments and the camera arm 301B which grips the medical equipment serve as moving elements (i.e., slaves). When the operator O operates the operating handle 201, the movement of the instrument arm 301A or the camera arm 301B is controlled so that the distal end portion of each instrument arm 301A (i.e., the end effector of the instrument 301a) or the distal end portion of the camera arm 301B (i.e., the endoscope 301b) will trace the movement of the operating handle 201 and shift accordingly.

The slave manipulator 300 is configured to control the movements of the respective instrument arms 301A according to a movement scale factor which has been set. For example, in a case in which the movement scale factor has been set to be 1/2, the end effector of the instrument 301a is controlled to shift by one half (1/2) of a distance by which the operating handle 201 has shifted. As a result, precise surgery can be accurately performed.

As illustrated in FIGS. 3 to 6, the operating handle 201 includes a master operation main body 210, a first operating portion 220, a second operating portion 230, and a grip 240.

The master operation main body 210 is arranged at a distal end of the master operation input device 200 (the operating handle 201) as viewed from a fixed end of the master operation input device 200. The master operation main body 210 has an elongated shape extending in a direction of roll axis (i.e., A direction). Specifically, the master operation main body 210 is in the elongated substantially cylindrical shape. An end portion, toward the fixed end (in A1 direction), of the master operation main body 210 is supported by a distal end of the arm portion 201a of the operating handle 201 so as to be capable of rotating about the roll axis (i.e., the A direction). The master operation input device 200 is configured to control the surgical instrument such that the surgical instrument rotates about a roll axis when the master operation main body 210 is operated to rotate about the roll axis. A connecting portion 211 for connecting the grip 240 is provided at an end portion, toward the distal end (in A2 direction), of the master operation main body 210.

In the first embodiment, the master operation input device 200 is configured to provide a command value as to the position and orientation of the surgical instrument of the slave manipulator 300 in response to a change in the position and orientation of the master operation main body 210 (the operating handle 201) by the operator O. That is, the master operation input device 200 is configured to control the position and orientation of the surgical instrument of the slave manipulator 300 in conjunction with the position and orientation of the master operation main body 210 (the operating handle 201). This configuration allows intuitive operation of the surgical instrument by the operator O using the master operation main body 210.

To enable opening and closing operations, the first operating portion 220 and the second operating portion 230 are supported on the master operation main body 210 so as to be capable of pivoting about the yaw axis (i.e., C direction) with respect to the master operation main body 210. In the closed position, both of the first and second operating portions 220 and 230 are in the elongated shape extending in the direction of the roll axis (i.e., the A direction). Specifically, the first and second operating portions 220 and 230 are in the elongated plate-like shape.

The first operating portion 220 is arranged on one side (toward B1 direction) of the master operation main body 210 in a direction of pitch axis (i.e., B direction). The second operating portion 230 is arranged on the other side (toward B2 direction) of the master operation main body 210 in the direction of pitch axis. That is, the first and second operating portions 220 and 230 are arranged at positions opposed to each other with respect to the master operation main body 210 in the direction of pitch axis.

To perform the opening operation, both of the first and second operating portions 220 and 230 move toward the master operation main body 210. To perform the closing operation, both of the first and second operating portions 220 and 230 move away from the master operation main body 210. The first and second operating portions 220 and 230 are intended to open and close the surgical instrument that can be opened and closed, such as a grasping forceps.

The first and second operating portions 220 and 230 are configured to be operable by first and second fingers of the operator O. The first and second fingers of the operator O may be, for example, the thumb and forefinger of the operator O. Alternatively, the first and second fingers of the operator O may be, for example, the thumb and middle finger of the operator O. Each of the first and second operating portions 220 and 230 has a finger insertion ring 250. The finger insertion rings 250 allow the fingers of the operator O to be held on the first and second operating portions 220 and 230, and therefore contribute to reliable opening and closing operations by the first and second operating portions 220 and 230. Each of the finger insertion rings 250 has substantially an annular shape so that the finger of the operator O can be inserted therein. The finger insertion ring 250 of the first operating portion 220 is for inserting the first finger of the operator O. The finger insertion ring 250 of the second operating portion 230 is for inserting the second finger of the operator O.

In the first embodiment, the grip 240 is configured to be attachable and detachable to and from a distal end (toward A2 direction) of the master operation main body 210. Thus, those operators O who feel it easy to operate the device with the grip 240 may attach the grip 240 to the master operation main body 210 during the operation. On the other hand, those operators O who feel it easy to operate the device without the grip 240 may detach the grip 240 from the master operation main body 210 during the operation. That is, usability of the operating handle 201 can be changed according to individual operators. In general, the master operation input device 200 is used by various operators O. Thus, the master operation input device 200 capable of changing its usability according to individual operators O is very effective.

In the first embodiment, the grip 240 is configured to be gripped with the fingers other than the first and second fingers of the operator O in a state in which the grip 240 is attached to the master operation main body 210. That is, the grip 240 can be easily gripped with the fingers not involved in the operation of the first and second operating portions 220 and 230. For example, the grip 240 is gripped with the middle finger, ring finger, and little finger of the operator O.

In the first embodiment, the connecting portion 211 of the master operation main body 210 has an engagement portion 211a. The grip 240 has an engagement portion 241 which detachably engages with the engagement portion 211a. That is, the grip 240 is attachable and detachable by the simple configuration. The engagement portion 211a of the connecting portion 211 of the master operation main body 210 is configured as a groove. The engagement portion 241 of the grip 240 is configured as a projection having a shape corresponding to the groove. The engagement portion 211a configured as the groove and the engagement portion 241 configured as the projection are fitted to each other, and thus engage with each other. The engagement portion 211a and the engagement portion 241 are examples of a "first engagement portion" and a "second engagement portion" recited in the claims, respectively.

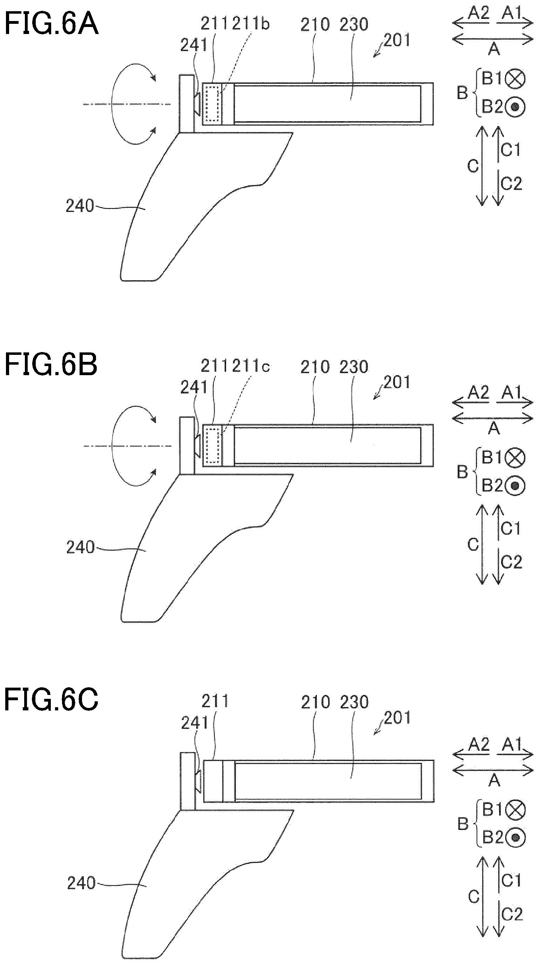

As illustrated in FIG. 6A, the connecting portion 211 of the master operation main body 210 may include, for example, a built-in bearing 211b having substantially no frictional force. The connecting portion 211 is configured to connect the master operation main body 210 and the grip 240 so that the master operation main body 210 and the grip 240 are capable of rotating about the roll axis (i.e., the A direction) by the bearing 211b independently from each other. In this case, when the grip is released, the grip 240 moves from a rotated position to a vertically-oriented position due to its own weight because the bearing 211b does not have a frictional force.

As illustrated in FIG. 6B, the connecting portion 211 of the master operation main body 210 may include, for example, a built-in bearing 211c having a frictional force. The connecting portion 211 is configured to connect the master operation main body 210 and the grip 240 so that the master operation main body 210 and the grip 240 are capable of rotating about the roll axis (i.e., the A direction) by the bearing 211c independently from each other. In this case, since the bearing 211c has a frictional force, the grip 240 remains at a rotated position due to the frictional force of the bearing 211c even when the grip is released.

As illustrated in FIG. 6C, the connecting portion 211 of the master operation main body 210 may include no built-in bearing, for example. In this case, the connecting portion 211 is configured to connect the master operation main body 210 and the grip 240 so that the master operation main body 210 and the grip 240 rotate about the roll axis (i.e., the A direction) in conjunction with each other. That is, the grip 240 is fixed to the master operation main body 210 such that the grip 240 can rotate together with the master operation main body 210 but that the grip 240 cannot rotate with respect to the master operation main body 210. In this case, the master operation main body 210 and the grip 240 remain at a rotated position even when the grip is released. The finger insertion rings 250 are not illustrated in FIGS. 6A to 6C.

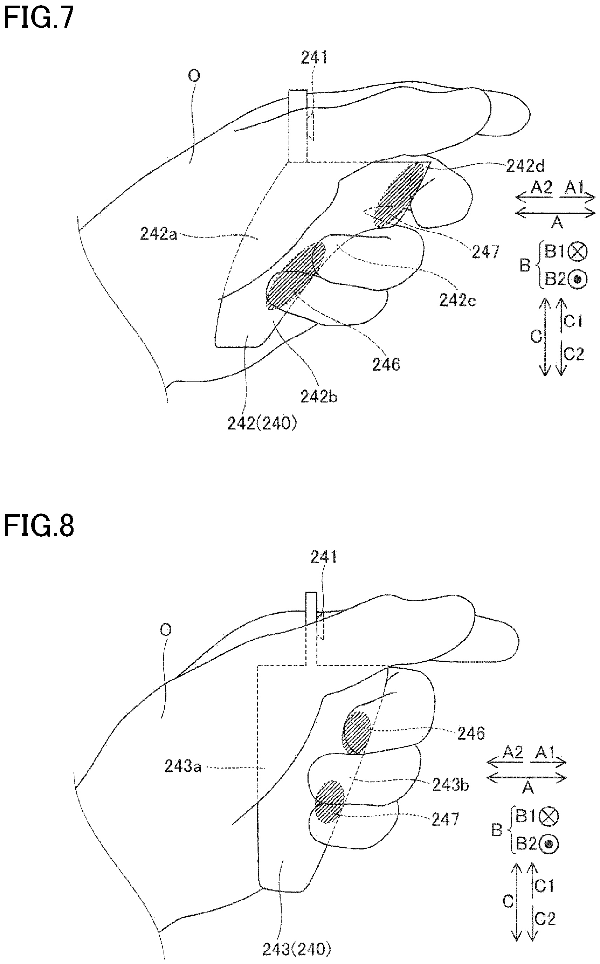

In the first embodiment, as illustrated in FIGS. 7 to 10, the grip 240 includes a plurality of grips (four grips) 242 to 245 having different shapes. That is, the plurality of grips 242 to 245 having different shapes are prepared for use in the master operation input device 200. Each of the plurality of grips 242 to 245 is configured to be attachable and detachable to and from the master operation main body 210. The operator O can select a grip of the operator's preference from among the plurality of grips 242 to 245, which further enhances the flexibility of change in the usability according to individual operators O. For the convenience of illustration, FIGS. 3 to 6 illustrate examples in which the grip 242 is attached to the master operation main body 210. The grips 243 to 245 may also be attached to the master operation main body 210.

As illustrated in FIG. 7, the grip (a trigger grip) 242 has an elongated shape in the vertical direction (C direction). The master operation main body 210 and the grip 242 form a gun-like shape (a pistol-like shape) as a whole in a state in which the grip 242 is attached to the master operation main body 210. This shape achieves firm gripping of the grip 242, giving the operator O an assured sense of gripping.

The grip 242 is configured to be gripped with the palm, middle finger, ring finger, and little finger of the operator O. Specifically, the grip 242 is configured such that a rear surface 242a is gripped with the palm of the operator O, and that a front surface 242b is gripped with the middle finger, ring finger, and little finger of the operator O. The rear surface 242a curves so as to protrude rearward (in the A2 direction) when viewed from the side (as viewed in the B direction). The front surface 242b includes a first portion 242c which curves so as to recess rearward, and a second portion 242d formed above (i.e., toward the C1 direction of) the first portion 242c and protrudes forward (in the A1 direction) from the first portion 242c. The first portion 242c is gripped with the ring finger and little finger of the operator O. The second portion 242d is gripped with the middle finger the operator O. The grip 242 is gripped mostly with the operator O's middle finger which grips the second portion 242d, and the ring finger and little finger of the operator O which grip the first portion 242c are movable relatively freely.

The grip 242 has a switch 246 for locking the surgical instrument. Since the surgical instrument can be locked by the switch 246 at hand, the surgical instrument can be immediately locked when the surgical instrument needs to be locked during surgery. The switch 246 is disposed at the first portion 242c of the grip 242 which is the portion gripped with the ring finger and little finger of the operator O. The switch 246 is configured to lock the surgical instrument when the switch 246 is turned on. Once locked, the surgical instrument does not move even if the operator O operates the first operating portion 220 and the second operating portion 230. The switch 246 is configured to unlock the surgical instrument when the switch 246 is turned off. Once unlocked, the surgical instrument moves in response to the operation of the first operating portion 220 and the second operating portion 230 by the operator O. The switch 246 functions as a clutch switch for switching between locking and unlocking the surgical instrument. Examples of the switch 246 include a button switch and a slide switch.

The grip 242 also has a contact sensor 247 for detecting whether the operator O is operating the device or not. The contact sensor 247 is disposed at the second portion 242d of the grip 242 which is the portion gripped with the middle finger of the operator O. The contact sensor 247 is configured to lock the surgical instrument when the contact sensor 247 does not detect that the operator O is operating the device. This configuration can prevent the surgical instrument from moving unintentionally when the operator O does not grip the grip 242 and is not operating the device. The contact sensor 247 is configured to unlock the surgical instrument when the contact sensor 247 detects that the operator O is operating the device. This configuration allows the surgical instrument to move when the operator O grips the grip 242 and is operating the device. Examples of the contact sensor 247 include a capacitance type sensor and a pressure sensitive sensor.

As illustrated in FIG. 8, the grip (straight grip) 243 has an elongated shape in the vertical direction (C direction). The master operation main body 210 and the grip 243 form a gun-like shape (a pistol-like shape) as a whole in a state in which the grip 243 is attached to the master operation main body 210. This shape achieves firm gripping of the grip 243, giving the operator O an assured sense of gripping.

The grip 243 is configured to be gripped with the palm, middle finger, ring finger, and little finger of the operator O. Specifically, the grip 243 is configured such that a rear surface 243a is gripped with the palm of the operator O, and that a front surface 243b is gripped with the middle finger, ring finger, and little finger of the operator O. The rear surface 243a extends substantially straight in the vertical direction (in the C direction) when viewed from the side. The front surface 243b curves so as to protrude forward (in the A1 direction) when viewed from the side. The grip 243 is gripped mostly with the ring finger and little finger of the operator O, and the middle finger of the operator O is movable relatively freely.

In the case of the grip 243, the switch 246 is disposed at a portion of the front surface 243b of the grip 243 which is gripped with the middle finger of the operator O. In the case of the grip 243, the contact sensor 247 is disposed at a portion of the front surface 243b of the grip 243 which is gripped with the ring finger and little finger of the operator O.

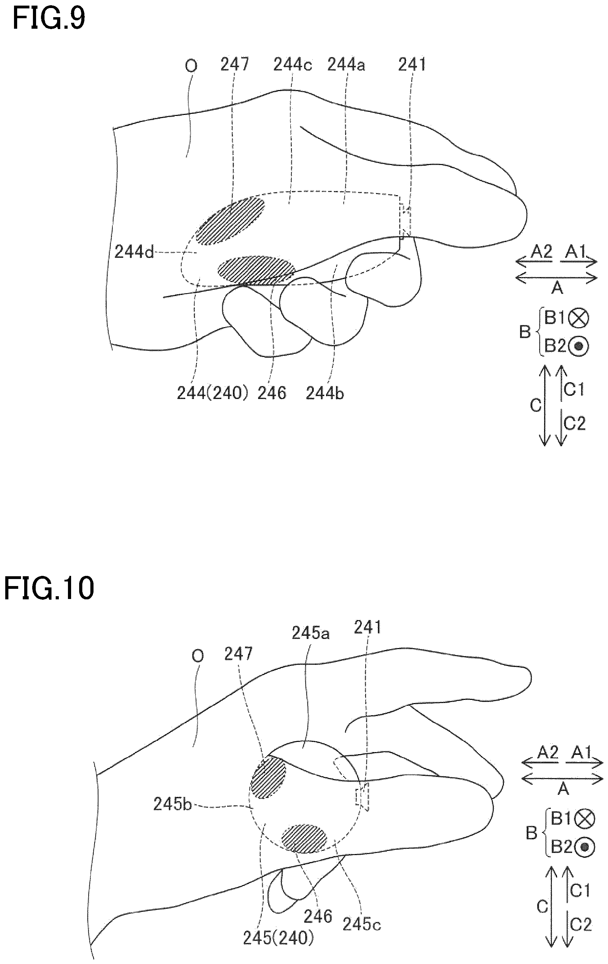

As illustrated in FIG. 9, the grip (tong grip) 244 has an elongated shape in the front-rear direction (the B direction). This shape of the grip 244 allows the operator O to grip the grip 244 while keeping the hand relatively horizontal.

The grip 244 is configured to be gripped with the palm, middle finger, ring finger, and little finger of the operator O. Specifically, the grip 244 is configured such that an upper surface 244a is gripped with the palm of the operator O, and that a lower surface 244b is gripped with the middle finger, ring finger, and little finger of the operator O. The upper surface 244a includes a first portion 244c which extends substantially straight in the front-rear direction (B direction), and a second portion 244d formed behind (i.e., toward the A2 direction of) the first portion 244c and curves so as to protrude rearward from the first portion 244c. The lower surface 244b extends substantially straight in approximately the front-rear direction when viewed from the side. The grip 244 is gripped mostly with the ring finger and little finger of the operator O, and the middle finger of the operator O is movable relatively freely.

In the case of the grip 244, the switch 246 is disposed at a portion of the lower surface 244b of the grip 244 which is gripped with the ring finger and little finger of the operator O. In the case of the grip 244, the contact sensor 247 is disposed at a portion of the upper surface 244a of the grip 244 which is gripped with the palm of the operator O.

As illustrated in FIG. 10, the grip (ball grip) 245 has a spherical shape. This shape of the grip 245 gives the operator O a uniform sense of gripping as if the whole palm is covering the ball.

The grip 245 is configured to be gripped with the palm, ring finger, and little finger of the operator O. Specifically, the grip 245 is configured such that an upper surface 245a and a rear surface 245b are gripped with the palm of the operator O, and that a lower surface 245c is gripped with the ring finger and little finger of the operator O. The grip 245 is gripped mostly with the ring finger and little finger of the operator O, and the middle finger of the operator O is freely movable. Thus, in the case of using the grip 245, the thumb and middle finger of the operator O can operate the first operating portion 220 and the second operating portion 230.

In the case of the grip 245, the switch 246 is disposed at a portion of the lower surface 245c of the grip 245 which is gripped with the ring finger and little finger of the operator O. In the case of the grip 245, the contact sensor 247 is disposed at a portion of the rear surface 245b of the grip 245 which is gripped with the palm of the operator O.

With respect to the grips 242 to 245, the portion gripped with the palm of the operator O is made of a material (a cushion material) softer than a material used for the portion gripped with the fingers of the operator O. In other words, with respect to the grips 242 to 245, the portion gripped with the fingers of the operator O is made of a material harder than the material used for the portion gripped with the palm of the operator O.

As illustrated in FIGS. 1 and 2, the operation pedal unit 202 includes pedals that can be operated by the foot of the operator O. To each pedal, a certain function may be assigned. In some function, a switching command can be input so that the target to be controlled by the operating handle 201 is changed among the plurality of instrument arms 301A and the camera arm 301B. When a field of view needs to be changed during surgery, the operator O operates the operation pedal unit 202 to change the target to be controlled by the operating handle 201 from the instrument arm 301A to the camera arm 301B. The endoscope 301b is thus movable through the operation of the operating handle 201. After moving the endoscope 301b, the operator O operates the operation pedal unit 202 again so that the target to be operated by the operating handle 201 is back to the instrument arm 301A from the camera arm 301B, thereby making it possible to continue the surgery. The operation pedal unit 202 is arranged at a lower position so as to be operable by the foot. The operation pedal unit 202 is configured to be movable in the Y direction.

As another function of the operation pedal unit 202, a command as to the movement of the instrument 301a attached to the distal end of the instrument arm 301A can be input. For example, the operation pedal unit 202 can input a command to cut or coagulate a surgical site with the instrument 301a. For example, a voltage for cutting or a voltage for coagulation may be applied to the instrument 301a through the operation of the operation pedal unit 202.

As still another function of the operation pedal unit 202, a command to lock the surgical instrument can be input. For example, the surgical instrument can be locked through the operation of the operation pedal unit 202. For example, the surgical instrument can be unlocked through the operation of the operation pedal unit 202.

The display unit 203 can display the images taken by the endoscope 301b. The display unit 203 includes a scope type display unit 203a or a non-scope type display unit 203 b (see FIG. 2). The scope type display unit 203a is, for example, a display unit which an operator looks into. The non-scope type display unit 203b represents a concept that includes an open display unit having a flat screen which an operator does not look into, just like a display of a common personal computer. Further, the scope type display unit 203a and the non-scope type display unit 203b are configured to be selectively attachable to the master operation input device 200. FIG. 1 illustrates an example in which the scope type display unit 203a is attached to the master operation input device 200. The scope type display unit 203a or the non-scope type display unit 203b is configured to be supported by the display unit supporting arm 204. Thus, both of an immersive master operation input device and an open master operation input device are selectable, and the master operation input device 200 having flexibility in terms of the display unit 203 can be provided.

Surgery generally takes several hours, and a surgeon (the operator O) may feel isolated if the surgeon works for a long time with an immersive master operation input device. In such a case, the master operation input device can be switched to an open type before or during the surgery so that the surgeon can feel that the surgeon is performing the surgery as one of team members.

If the master operation input device has flexibility and extensibility in terms of the display unit, the display unit can be repaired independently of the device even when the display unit fails or is damaged. This is advantageous because the whole device does not need to be replaced. As another advantage, the display unit can be upgraded every time the display unit with improved definition and quality is developed, without replacing the whole device. As still another advantage, the operator can select the display unit depending on the operator's preferred manufacturer and specification (such as size, shape, and an operation panel).

The display unit 203 includes a terminal 203 c. Examples of the terminal 203c include a terminal capable of transmitting video, such as a serial digital interface (SDI) terminal, an analog component terminal, a High-Definition Multimedia Interface (HDMI (registered trademark)) terminal, and a universal serial bus (USB) terminal. The terminal 203c is connected to the control device 206. Specifically, when a connecting wire is connected to the terminal 203c, image information is transmitted from the control device 206 to the display unit 203. When the connecting wire is disconnected from the terminal 203c, the display unit 203 can be detached from the master operation input device 200.

In a case in which the scope type display unit 203a is attached, a 3D image taken by the endoscope 301b gripped by the camera arm 301B of the slave manipulator 300 is displayed. Also in a case in which the non-scope type display unit 203b is attached, a 3D image taken by the endoscope 301b provided in the slave manipulator 300 is displayed. In the case in which the non-scope type display unit 203b is attached, a 2D image taken by the endoscope 301b provided in the slave manipulator 300 may be displayed.

The scope type display unit 203a is a viewer which the operator O looks into. The scope type display unit 203a displays an image for the right eye of the operator O and an image for the left eye of the operator O. The scope type display unit 203a is, for example, a stereoscope. That is, the display of the scope type display unit 203a includes a left-eye display and a right-eye display. When the operator looks into the display of the scope type display unit 203a, the left eye cannot see the right-eye display, and the right eye cannot see the left-eye display. The display of the scope type display unit 203a may be comprised of a liquid crystal display or an organic EL display. The display of the scope type display unit 203a may be a projection type display.

The non-scope type display unit 203b is an open display unit which the operator can see without looking into it. The non-scope type display unit 203b is a direct view type display unit. That is, the display of the non-scope type display unit 203b has a screen with a flat or curved surface. For example, a display having a diagonal line of 10 to 90 inches may be used as the display of the non-scope type display unit 203b. However, taking the balance between the sufficient viewability of the surgical field and the ease of replacement of the display into consideration, a display of about 15 to 35 inches is suitable. The display of the non-scope type display unit 203b may be comprised of a liquid crystal display or an organic EL display. The display of the non-scope type display unit 203b may be a projection type display. In order that the operator O can three-dimensionally see the image taken by the endoscope 301b, any known stereoscopic system using polarizing glasses or active shutter glasses may be applied.

The display unit supporting arm 204 is configured to support the display unit 203. The display unit supporting arm 204 is arranged so as to oppose to the operator O with respect to the display unit 203. That is, the display unit supporting arm 204 is arranged behind the display unit 203 (toward the Y2 direction).

The arm rest 205 is configured to support the arms of the operator O. The arm rest 205 is arranged forward (toward the Y1 direction) of the operating handle 201 and supports the arms of the operator O. This configuration can stabilize the arms of the operator O, and therefore allows the operator O to stably control the operating handle 201. Specifically, even in the case where a fine movement of the end effector is required, the operator O can stably operate the end effector with the elbows resting on the arm rest 205. This can reduce the burden of the operator O even if the surgery takes long time. The arm rest 205 is supported by the support mechanism 209.

As illustrated in FIG. 4, the control device 206 includes, for example, the control unit 261 including an arithmetic unit such as a CPU, a storage unit 262 including a memory such as a ROM and a RAM, and an image control unit 263. The control device 206 may be configured as a single controller which provides centralized control, or may be configured as a plurality of controllers which work in cooperation with each other and provide distributed control. The control unit 261 determines whether the movement type instruction input through the operating handle 201 is a movement type instruction to be executed by the instrument arm 301A, or a movement type instruction to be executed by the endoscope 301b, in accordance with the state of the operation pedal unit 202. If the control unit 261 determines that the movement type instruction input through the operating handle 201 is a movement type instruction to be executed by the instrument 301a, the control unit 261 transmits this movement type instruction to the instrument arm 301A. In response, the instrument arm 301A is driven, as a result of which the operation of the instrument 301a attached to the instrument arm 301A is controlled.

Alternatively, if the control unit 261 determines that the movement type instruction input through the operating handle 201 is a movement type instruction to be executed by the endoscope 301b, the control unit 261 transmits the movement type instruction to the camera arm 301B. In response, the camera arm 301B is driven, as a result of which the operation of the endoscope 301b attached to the camera arm 301B is controlled.

The storage unit 262 stores, for example, control programs corresponding to the types of the instruments 301a. The control unit 261 reads the control program in accordance with the type of the instrument 301a attached, thereby allowing the movement instruction entered through the operating handle 201 and/or operation pedal unit 202 of the master operation input device 200 to instruct each instrument 301a to perform a suitable operation.

The image control unit 263 transmits images taken by the endoscope 301b to the terminal 203c of the display unit 203. The image control unit 263 processes or corrects the image as needed.

The posture changer 208 is configured to receive an instruction to move the operating handle 201, the display unit 203 supported by the display unit supporting arm 204, and the arm rest 205, in the vertical direction. The posture changer 208 is configured to receive an instruction to transform the master operation input device 200 between a first posture (a standing position) and a second posture (a seated position). That is, the posture changer 208 is an operating unit configured to receive a posture change instruction to change the posture of the master operation input device 200 between the standing position (the first posture) and the seated position (the second posture). The posture changer 208 includes a plurality of operation buttons.

The support mechanism 209 supports the operating handle 201 and the arm rest 205. Further, the support mechanism 209 supports the display unit 203 via the display unit supporting arm 204. The support mechanism 209 is configured to move the operating handle 201, the arm rest 205, and the display unit 203 supported by the display unit supporting arm 204 in the vertical direction. The posture of the support mechanism 209 may be changed by a driving section using air pressure or oil pressure, or may be manually changed by the operator O or any other person.

The controller 400 is configured to perform control to drive a plurality of joints of the slave manipulator 300, based on the operation of the master operation input device 200 by the operator O. The controller 400 includes, for example, an arithmetic unit such as a CPU, and a memory such as a ROM and a RAM

### [Second Embodiment]

Now, a second embodiment of the present invention will be described with reference to FIGS. 1, 2, 11 and 12. Unlike the first embodiment, in which the grip is directly attached to the master operation main body, the second embodiment is an example in which the grip is attached to the master operation main body via a joint portion. Note that the same reference characters are used in the drawings to designate the same elements as those of the first embodiment, and explanation thereof will be omitted.

### (Configuration of Master Operation Input Device)

As illustrated in FIGS. 1 and 2, the master operation input device 600 of the second embodiment differs from the master operation input device 200 of the first embodiment in that the master operation input device 600 has an operating handle 601.

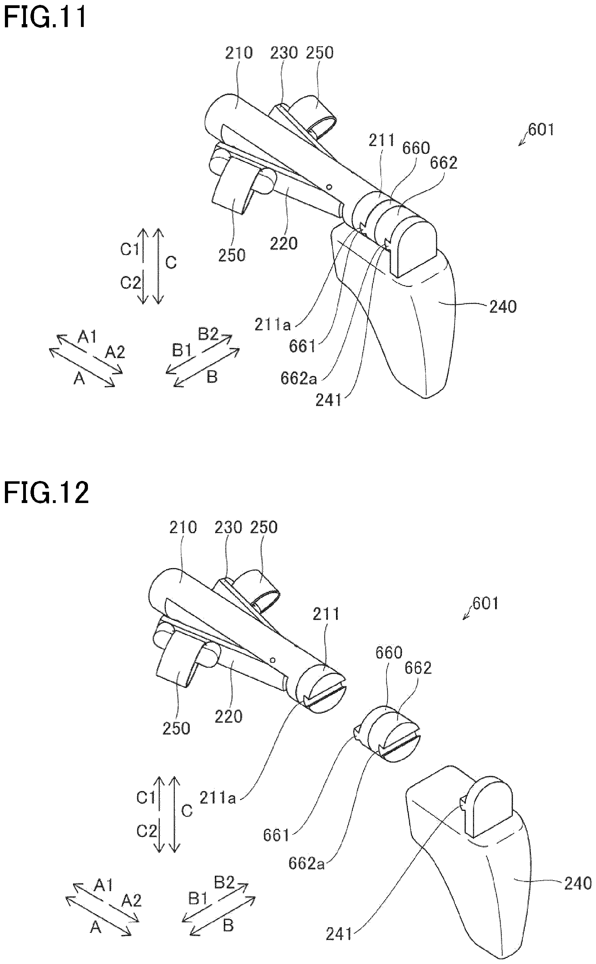

As illustrated in FIGS. 11 and 12, the operating handle 601 of the second embodiment has a joint portion 660 configured to be attachable and detachable to and from the distal end of the master operation main body 210, and capable of rotating about the roll axis (the A direction) with respect to the master operation main body 210 in a state in which the operating handle 601 is attached to the master operation main body 210. The grip 240 is attached to the joint portion 660. That is, the grip 240 of the second embodiment is detachably attached to the master operation main body 210 via the joint portion 660. This configuration allows the grip 240 to rotate about the roll axis independently of the master operation main body 210. The grip 240 of the second embodiment is attachable and detachable to and from the joint portion 660. It is therefore possible to choose as necessary whether to attach the joint portion 660 and the grip 240 to the master operation main body 210, or whether to attach only the grip 240 to the master operation main body 210 without the joint portion 660. If a plurality of grips 240 are provided, the joint portion 660 is provided for each grip 240.

The joint portion 660 includes an engagement portion 661 which detachably engages with the engagement portion 211a of the connecting portion 211 of the master operation main body 210, and a movable portion 662 capable of rotating about the roll axis with respect to the master operation main body 210. The engagement portion 661 of the joint portion 660 is configured as a projection having a shape corresponding to the engagement portion 211a configured as a groove. The engagement portion 211a configured as the groove and the engagement portion 661 configured as the projection are fitted to each other, and thus engage with each other. The movable portion 662 has an engagement portion 662a. In the second embodiment, the engagement portion 241 of the grip 240 is configured to detachably engage with the engagement portion 662a. The engagement portion 662a of the movable portion 662 of the joint portion 660 is configured as a groove. The engagement portion 662a configured as the groove and the engagement portion 241 configured as the projection are fitted to each other, and thus engage with each other.

The master operation input device 600 of the second embodiment is configured to control the surgical instrument such that the surgical instrument rotates about the roll axis when the grip 240 is operated to rotate about the roll axis (in the A direction) with respect to the master operation main body 210 via the joint portion 660 attached to the master operation main body 210. That is, the master operation input device 600 is configured to control the surgical instrument such that the surgical instrument rotates about the roll axis in conjunction with the rotating of the grip 240 and the joint portion 660 about the roll axis. This configuration allows the operator O to rotate the surgical instrument about the roll axis by simple and intuitive operation.

The other configurations of the second embodiment are the same as, or similar to, those of the first embodiment.

### [Third Embodiment]

Now, a third embodiment of the present invention will be described with reference to FIGS. 1, 2, 13 and 14. Unlike the first embodiment, in which the grip is directly attached to the master operation main body, the third embodiment is an example in which the grip is attached to the master operation main body via an engagement portion. Note that the same reference characters are used in the drawings to designate the same elements as those of the first embodiment, and explanation thereof will be omitted.

### (Configuration of Master Operation Input Device)

As illustrated in FIGS. 1 and 2, the master operation input device 700 of the third embodiment differs from the master operation input device 200 of the first embodiment in that the master operation input device 700 has an operating handle 701.

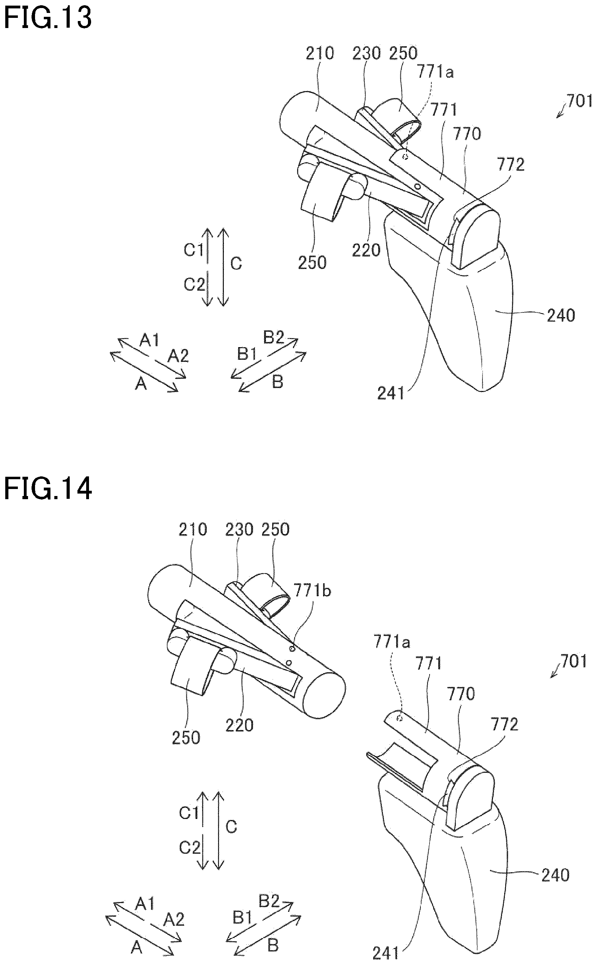

As illustrated in FIGS. 13 and 14, the operating handle 701 of the third embodiment has an engagement member 770 configured to be attachable and detachable to and from the distal end of the master operation main body 210. The grip 240 is attached to the engagement member 770. That is, the grip 240 of the third embodiment is detachably attached to the master operation main body 210 via the engagement member 770. The grip 240 is attachable and detachable to and from the engagement member 770.

The engagement member 770 includes an engagement portion 771 which engages with the distal end of the master operation main body 210, and an engagement portion 772 which engages with the grip 240. The engagement portion 771 is fitted to an outer surface of the master operation main body 210, and thus engages with the master operation main body 210. The engagement portion 771 has, on its inner surface, an engagement portion 771a configured as a projection. The master operation main body 210 has, in its outer surface, an engagement portion 771b configured as a groove having a shape corresponding to the engagement portion 771a configured as the projection. The engagement portion 771a configured as the projection and the engagement portion 771b configured as the groove are fitted to each other, and thus engage with each other. The engagement portion 772, which is configured as a groove, is fitted with, and thus engages with, the engagement portion 241 configured as a projection.

The other configurations of the third embodiment are the same as, or similar to, those of the first embodiment.

### [Variations]

The embodiments disclosed herein are meant to be illustrative in all respects and should not be construed to be limiting in any manner. The scope of the present invention is defined not by the above-described embodiments, but by the scope of claims, and includes all modifications (variations) within equivalent meaning and scope to those of the claims.

For example, it has been described in the first to third embodiments that four types of grips are prepared. However, this is not limiting. In the present invention, a plurality of types of grips other than four types may be prepared, or only one type of grip may be prepared.

It has been described in the first to third embodiments that the grip includes a plurality of grips having different shapes. However, this is not limiting. In the present invention, the grip may include a plurality of grips having different sizes. Alternatively, the grip may include both a plurality of grips having different sizes and a plurality of grips having different shapes.

Four types of shapes (the shapes shown in FIGS. 7 to 10) have been described as the shape of the grip in the first to third embodiments. However, this is not limiting. In the present invention, the grip may have a shape other than those shapes.

It has been described in the first to third embodiments that the master operation main body includes an engagement portion configured as a groove, and that the grip includes an engagement portion configured as a projection. However, this is not limiting. In the present invention, the master operation main body and the grip may include any type of engagement portion as long as the grip can be detachably attached to the master operation main body. For example, the master operation main body and the grip may include engagement portions such as a pawl and a hole.

It has been described in the first to third embodiments that the grip is provided with a switch and a contact sensor. However, this is not limiting. In the present invention, the grip may be provided with only one of the switch or the contact sensor, or both of the switch and the contact sensor may not be provided.

It has been described in the first to third embodiments that a portion of the grip gripped with the palm of the operator is made of a material softer than a material used for a portion of the grip gripped with the fingers of the operator. However, this is not limiting. In the present invention, the portion of the grip gripped with the palm of the operator and the portion of the grip gripped with the fingers of the operator may be made of the same material.

It has been described in the first to third embodiments that a finger insertion ring is provided for each of the first operating portion and the second operating portion. However, this is not limiting. In the present invention, the finger insertion ring does not have to be provided.

### DESCRIPTION OF REFERENCE CHARACTERS

100: Master-Slave Manipulator
200, 600, 700: Master Operation Input Device
210: Master Operation Main Body
211a: Engagement Portion (First Engagement Portion)
220: First Operating Portion
230: Second Operating Portion
240, 242, 243, 244, 245: Grip
241: Engagement Portion (Second Engagement Portion)
246: Switch
247: Contact Sensor
250: Finger Insertion Ring
300: Slave Manipulator
400: Controller
660: Joint Portion

## Claims

1. A master operation input device for operating a slave manipulator having a surgical instrument as an end effector, the master operation input device comprising:
a master operation main body arranged at a distal end of the master operation input device as viewed from a fixed end of the master operation input device;
a first operating portion and a second operating portion which are supported on the master operation main body so as to be capable of pivoting with respect to the master operation main body to enable opening and closing operations; and
a grip configured to be attachable and detachable to and from a distal end of the master operation main body.

2. The master operation input device of claim 1, wherein
the first operating portion and the second operating portion are configured to be operable by a first finger and a second finger of an operator, and
the grip is configured to be gripped with fingers other than the first finger and the second finger of the operator in a state in which the grip is attached to the master operation main body.

3. The master operation input device of claim 1 or 2, wherein
each of the first operating portion and the second operating portion includes a finger insertion ring.

4. The master operation input device of any one of claims 1 to 3, wherein
the master operation input device is configured to provide a command value as to a position and an orientation of the surgical instrument of the slave manipulator in response to a change in the position and the orientation of the master operation main body by the operator.

5. The master operation input device of any one of claims 1 to 4, wherein
the master operation main body includes a first engagement portion, and
the grip includes a second engagement portion which detachably engages with the first engagement portion.

6. The master operation input device of any one of claims 1 to 5, wherein
the grip includes a plurality of grips different in at least one of size or shape, and
each of the plurality of grips is configured to be attachable and detachable to and from the master operation main body.

7. The master operation input device of claim 6, wherein
the master operation main body is in an elongated shape, and
the master operation main body and the grip form a gun-like shape as a whole in a state in which the grip is attached to the master operation main body.

8. The master operation input device of claim 7, wherein
a front surface of the grip curves so as to protrude forward when viewed from side.

9. The master operation input device of claim 7, wherein
a front surface of the grip curves so as to recess rearward when viewed from side.

10. The master operation input device of claim 6, wherein
the master operation main body is in an elongated shape, and
the grip has a spherical shape.

11. The master operation input device of any one of claims 1 to 10, wherein
the grip includes a switch for locking the surgical instrument.

12. The master operation input device of any one of claims 1 to 11, wherein
the grip includes a contact sensor for detecting whether the operator is operating the master operation input device or not.

13. The master operation input device of any one of claims 1 to 12, comprising:
a joint portion configured to be attachable and detachable to and from the distal end of the master operation main body, and capable of rotating about a roll axis with respect to the master operation main body in a state in which the joint portion is attached to the master operation main body, wherein
the grip is attached to the joint portion.

14. The master operation input device of claim 13, wherein
the grip is configured to be attachable and detachable to and from the joint portion.

15. The master operation input device of claim 13 or 14, wherein
the master operation input device is configured to control the surgical instrument such that the surgical instrument rotates about a roll axis when the grip is operated to rotate about the roll axis with respect to the master operation main body via the joint portion attached to the master operation main body.

16. A master-slave manipulator, comprising:
a slave manipulator having a surgical instrument as an end effector and having a plurality of joints;
the master operation input device of any one of claims 1 to 15; and
a controller which performs control to drive the plurality of joints of the slave manipulator, based on an operation of the master operation input device by an operator.
